# EUROPEAN PATENT APPLICATION

(11) **EP 2 385 474 A1**
(43) Date of publication of application: **09.11.2011**
(21) Application number: 10162302.3
(22) Date of filing: 07.05.2010
(51) Int. Cl.: G06F 19/00

(54) **Method for analysing medical data**

(71) Applicant: TomTec Imaging Systems GmbH, 85716 Unterschleissheim (DE)
(72) Inventor: Baumann, Rolf, 88239, Wangen (DE)
(74) Representative: Schinkel, Reta

(57) **Abstract**

The invention relates to a method for analysing medical data in view of a specific clinical question, the method comprising the steps of a) providing one or several medical datasets comprising data acquired by means of one or several diagnostic modalities from one patient; b) providing a clinical question to be answered by the medical dataset(s); c) selecting one or several evaluation methods which are generally suitable for returning relevant information with regard to the clinical question; and d) automatically analysing the medical dataset(s) with regard to their suitability to be used for the one or several evaluation methods and calculating corresponding quality factors for each pair of medical dataset and evaluation method.

## Description

The invention relates to a method for analysing medical data, and in particular a method for selecting one or several evaluation methods for analysing medical data in view of a specific clinical question.

Due to the ongoing research and advances in the acquisition and analysis of clinical/medical data, in particular medical imaging data, a large number of evaluation methods has been developed in the past years. Thus, for each particular clinical question, a vast number of different evaluation methods are available.

In addition, it is becoming more and more common for clinical institutions such as hospitals to collect medical data in multi-modal platforms such as Picture Archiving and Communication Systems (PACS) such as a CVIS (Cardiovascular Information Solution) or a RIS (Radiology Information System) or a Hospital Information Systems (HIS). Thus, it is often the case that several types of medical datasets are collected from one patient. Since in addition, a varied spectrum of different evaluation methods is available, it has become difficult for the clinical practitioner to make proper use of the advanced technology which is now available.

According to the prior art, the medical practitioner has to decide from his own experience which evaluation method he or she is going to use for a specific clinical question, and on which medical datasets to use the evaluation method. Once he has made a decision, the medical dataset is loaded into the selected evaluation tool, with which it can be viewed and evaluated. In the following, the term "evaluation method" and "evaluation tool" are used interchangeably. Essentially, an evaluation tool is a software for evaluating medical data by a certain evaluation method and for returning respective results. Normally, the evaluation tool does not recognise whether a specific medical dataset is suitable for the particular evaluation method or not. Thus, the user can only judge from the display whether the medical dataset is suitable for this particular evaluation method. Or in the worst situation, the evaluation method when applied to the selected medical dataset yields a result which appears satisfactory, but which is in fact incorrect, or not comparable to the standards in the field, because the medical dataset was in fact not suitable for the selected evaluation method. For example, an evaluation of left ventricular ejection fraction from Ultrasound data by a single plane Simpson's method only works when the single plane is cutting through the center of the ventricle including apex and base. If the plane is off-centre, single plane Simpson's method can deliver misleading results.

Thus, at present, the user who wishes to evaluate medical datasets has to first choose an evaluation method, usually among several evaluation methods which are available for a particular clinical question. Then, he has to load one medical dataset after the other into the selected evaluation tool and view the data critically in order to see whether the evaluation method will yield a good result. This is time consuming and the quality of the results of the evaluation method depends largely on the skill of the user. This, in turn, has the consequence that the evaluation results can have high inter-observer variabilities and thus are difficult to compare to clinical standards. Thus, whether the right clinical diagnosis is found depends on the know-how of the user with regard to the different evaluation tools. For example, in one and the same hospital, the same clinical question could be tackled with different evaluation methods by different users. For example, different users might determine left ventricular ejection fraction by monoplane Simpson's method, biplane Simpson's method or a 3D method. With each evaluation method, the result will be an ejection fraction, but there will be no information on the quality of the evaluation result, i. e. the probability that the result is correct. Thus, some users may always choose the fastest method, which is usually not the most reliable.

In addition, the users have to be very well experienced and knowledgeable in the different evaluation methods and technologies for the different clinical questions. If such experience is missing, one often does not use the whole bandwidth of clinical information which is in fact available in the form of medical datasets for one patient.

It is an object of the invention to facilitate the selection of suitable evaluation techniques for a specific clinical question, and to improve the quality of the evaluation results.

This object is met by the method according to claim 1 comprising the following steps:
a) providing one or several medical datasets comprising data acquired by means of one or several diagnostic modalities from one patient;
b) providing a clinical question to be answered by the medical dataset(s);
c) selecting one or several evaluation methods which are generally suitable for returning relevant information with regard to the clinical question;
d) automatically analysing the medical dataset(s) with regard to their suitability to be used for the one or several evaluation methods and calculating corresponding quality factors for each pair of medical dataset and evaluation method.

In the first step, the user would select patient, and therewith the file containing all available medical datasets of that patient acquired by different diagnostic modalities. The diagnostic modalities may comprise imaging modalities such as Magnetic Resonance Imaging (MRI), Positron Emission Tomography (PET), Single Photon Emission Computed Tomography (SPECT), Computed Tomography (CT), ultrasound (e.g. B-mode, M-mode, Doppler of three-dimensional ultrasound), X-Rays or Infrared Imaging. In addition, the diagnostic modalities may comprise electromedical techniques such as electrocardiogram (ECG), or also simple diagnostic techniques such as blood pressure measurements or hemodynamic measurements. In an embodiment, the medical datasets of the particular patient may comprise different imaging datasets acquired by different imaging modalities, such as image datasets of the heart acquired by ultrasound and angiographic data acquired by X-Rays, in addition to an electrocardiogram and possible another 4D ultrasound dataset comprising a time sequence of 3-dimensional (3D) ultrasound images of the heart.

In the next step, the user has to select a clinical question, for example the left ventricular function.

Once the clinical question is defined, one or several evaluation methods are selected which are generally suitable for returning relevant information with regard to the clinical question. Preferably, this step is performed automatically by the system or device which is carrying out the invention and involves scanning the available evaluation tools for their suitability. To this end, each evaluation tool will be tagged with the clinical question(s) for which it may be used. Beforehand, the system might scan the relevant IT device for installed software. Alternatively, a table may be accessed where all available evaluation tools are listed and connected to the clinical questions for which they are relevant.

Once one or several suitable evaluation methods are identified, the medical datasets will be analysed with regard to their suitability to be used for the one or several evaluation methods, and corresponding quality factors will be calculated. This step is preferably performed fully automatic, i. e. without user input.

According to the invention, each possible combination or pair of evaluation method and medical dataset will be marked with a quality factor. The quality factor may for example take into consideration the quality of the medical dataset, the quality of the evaluation method (for example 3D methods are generally better reproducible than 2D methods), and/or further information which is not directly derived from the medical dataset or the evaluation method, but which may help in the decision whether the specific medical dataset is suitable for the specific evaluation method.

Optionally, the calculated quality factors are outputted, for example on a printer, a screen or a display, or by sending them electronically (e.g. by email) to the user.

The quality factors are then used to select at least one medical dataset as suitable to be evaluated by at least one evaluation method. The selection is usually done by choosing the pair of medical dataset and evaluation method with the highest quality factor. The selection may be fully automatic.

The invention covers two different ways of selecting: According to the first alternative, one evaluation method is selected for at least one of the medical datasets. This is generally done if several evaluation methods are available for at least one medical dataset. In this case, the inventive method has to select the best evaluation method.

According to another alternative, one evaluation method is already pre-selected as the preferred one. This may be advantageous if one clinical institution wants to make sure that all data are assessed by the same evaluation method, since there are often systematic differences in the results of different evaluation methods for the same clinical question. For example, a volume measurement of a heart chamber on MRI yields slightly different values to a measurement on ultrasound. In this case, the selection is in one of several medical datasets. In other words, several different datasets are analysed and the one having the best prerequisites for giving good results with the pre-selected preferred evaluation method is selected.

In an embodiment, the method comprises the further steps of :
g) evaluating the selected at least one medical dataset by the selected or preferred evaluation method;
h) outputting the result of the evaluation method; and
i) outputting the quality factor associated with the outputted result.

According to another embodiment, the selected evaluation method will not be automatically executed on the selected medical dataset. Rather, all calculated quality factors will be outputted. This gives the user the chance to select a suitable pair of medical dataset/ evaluation method, knowing the expected quality of the result. Thus, if two pairs give approximately similar quality factors, he may choose the second best evaluation method and medical dataset for reasons of personal preference. He may also decide to run both, or several of the pairs of evaluation method/medical dataset.

According to another embodiment, the method comprises the further steps of:
g) evaluating the selected at least one medical dataset by the preferred evaluation method and, in addition, by one or more of the other generally suitable evaluation methods;
h) outputting the results of each evaluation method; and
i) outputting the quality factor associated with each outputted result.

Thus, several or all possible evaluation methods are carried out on one or several, or all possible medical datasets. However, the associated quality factors are outputted together with each result, so that the user may choose to consider only such results with a good quality factor.

The evaluation methods may require user input, as is generally accepted for medical evaluation tools. Therefore, a mark given to the user for his skills in the respective evaluation methods may be incorporated into the quality factor. Such mark may for example be correlated to the number of years of experience of the user, or may be freely given by the clinical institution based on the user's experience.

The one or several medical datasets may for example be image datasets, such as two-dimensional (2D), three-dimensional (3D) or four-dimensional (4D) image datasets. The fourth dimension is time, i. e. a 4D image dataset is a time sequence of 3D datasets, for example to capture the contraction of the heart during the heart cycle. Such 4D image datasets of the heart can be acquired by ultrasound, MRI or X-Ray, in particular an interventional system such as a C-arm system. An image dataset may also be an angiographic or 2D or 3D CT, ultrasound or MRI image. Further available image datasets may be 2D or 3D stress echo datasets, which are medical images acquired during different workout levels of the heart.

The one or several medical datasets can also comprise non-image data, for example electromedical datasets such as electrocardiograms (ECG) or electroencephalogram (EEG) datasets. Further available medical datasets may be hemodynamic measurement values, blood pressure datasets or electric measurements of the heart.

The clinical question is preferably related to the cardiovascular system. In this case, it may for example be selected from the group comprising:
- determining the risk for a vascular disease;
- determining whether the patient has a coronary heart disease;
- determining the left ventricular function, e.g. the ejection fraction;
- determining whether an intraventricular dissynchrony is present.

The ventricular ejection fraction is defined as the difference between the maximum ventricular volume and the minimum ventricular volume divided by the maximum ventricular volume. For example, a left ventricular ejection fraction of 60% would be considered normal, while an ejection fraction of 30% would be considered diseased. To calculate the ejection fraction, end-systolic and end-diastolic ventricular volume must be estimated. Several evaluation methods are available for these measurements:

According to the single plane Simpson's method, one 2D image slice through the ventricle in the end-systolic and end-diastolic phases are used only. On such 2D images, the contour of the ventricle is defined (by hand or automatically), and the ventricle is assumed to be circular in cross-section for the volume calculation. The 2D image can for example be a 4-chamber view of the heart.

In biplane Simpson's method, two 2D images which are each oriented along the long axis of the ventricle, but at a right angle to each other, are used for determining the ventricular volumes. Again, the contour of the ventricle on each 2D image is determined, and the ventricle is assumed to be an ellipse in cross-section. Evidently, biplane Simpson's method is more precise.

Even more precise methods for determination of ventricular ejection fraction use 3D volume measurements, i. e. ventricular volume at end-diastole and end-systole is determined from a 3D image dataset of the heart.

The above is an example for three different evaluation methods which are in principle available for answering the same clinical question.

Another clinical question which may be posed is that of intra-ventricular dissynchrony. According to established medical guidelines, indications for intra-ventricular dissynchrony are a ventricular ejection fraction of < 35%, a lengthened QRS-complex in the electrocardiogram, and furthermore a low general quality of life, as determined by the NYHA (New York Heart Association) classification. Intra-ventricular dissynchrony can be treated by implanting a pacemaker.

In the case of this - or any other - clinical question, the system carrying out the inventive method may first ensure that all relevant medical datasets are available, for example the necessary image datasets for determining ventricular ejection fraction, and an ECG, and the NYHA class. In an embodiment of the invention, the system will output an alert if any one of the necessary medical datasets are missing.

In addition to the above described evaluation methods, the available evaluation methods may comprise:
- 2D evaluation methods for image datasets;
- 3D evaluation methods for image datasets;
- 4D evaluation methods for image datasets;
- measurement of blood flow;
- volume measurements of lesions or tumours;
- measurement of the synchrony of the movement of a heart chamber;
- measurement of the cross-section of a blood vessel;
- measurement of the intima-media thickness (IMT) of a blood-vessel;
- measurement of the degree of stenosis of a blood-vessel;
- measurement of muscle mechanics such as strain, displacement velocity, thickening.

The latter measurements may be used if the clinical question is coronary heart disease or generally a risk of vascular diseases.

Further possible evaluation tools may include Cardiac Performance Analysis, a 2D measurement of the synchrony of the heart chamber, or 4D LV-Analysis, a tool developed by the applicant Tomtec Imaging Systems GmbH, which allows a 3D measurement allowing assessment of the synchronicity of different parts of a ventricle wall. Another evaluation tool is "Q-Angio XA^{®}", a tool allowing measurements of the cross-section of coronary arteries. A further possible evaluation tool is the software package "M'ATH^{®}", which allows different measurements related to arteriosclerosis, for example the measurement of the thickness of the intima-media, but also of the grade of stenosis of a vessel or the elasticity of a vessel.

The analysis step (d) is preferably carried out automatically by the system. It usually involves at least two of the following principle steps:
- analysing the medical datasets;
- determining a merit factor associated with the evaluation tool;
- Taking into account further information (usually stored data), for example accessing a database of clinical a priori knowledge concerning the clinical question.

Concerning the first point mentioned above, the medical datasets may be analysed in a number of ways. In an embodiment, all available medical datasets are first pre-analysed to pre-select those medical datasets which are generally suitable for returning relevant information with regard to the clinical question. For example, the pre-analysis may comprise scanning the header data of all available medical datasets to find out what kind of images they are, i. e. with what modality they have been acquired (Ultrasound, CT, MRI), whether they are 2D, 3D or 4D data, and what body part they are showing. Such information is usually contained in the header of the data stored under the DICOM-standard. Alternatively, or in addition thereto, the pre-analysis step may comprise OCR (optical character recognition). This is relevant in particular for digitalised data, where the relevant information is written in the image margins and can be read out by OCR. In one embodiment, the information obtained by OCR or from the headers is used to filter the available medical datasets first, and only a sub-selection of the available medical datasets is thus identified and analysed further. In another embodiment, all available medical datasets are fully analysed with regard to their suitability to be used for the one or several evaluation methods.

The pre-selected (or all) medical datasets can be further analysed, for example by determining the content of a medical image dataset. This is generally difficult, of course, but in particular if the system knows from the header e.g. that the image shows the heart, it can find out by contour recognition whether a certain 2D image is a 2-chamber-view, a 3-chamber-view or a 4-chamber-view.

Another content-related information which can be automatically extracted is whether an image is native or contrast-enhanced. In contrast-enhanced images, the blood-vessels appear lighter. The content-recognition step may also be part of the pre-analysis.

Another step which may be carried out by the pre-analysis or analysis step is the determination of the quality of an image dataset in terms of noise level. This can for example be done by preparing and analysing a histogram of grey values of the image dataset. Thereby, different images showing essentially the same content can be compared and the best image selected.

Another possible analysis step is the determination of the contrast profile of a medical image dataset. Thereby, the image with the best contrast can be selected.

Further factors which may be extracted from the header, by OCR or by any other means, and which may be used in the pre-analysis or analysis step, are the acquisition mode (e.g. B-mode, M-mode, Doppler or 4D for ultrasound), the time resolution of a sequence of images, the spatial resolution of medical image datasets, or the field-of-view of an image. The field-of-view means the size and shape, but optionally also the position of the field-of-view within the body.

From the above-described analysis steps, one component of the quality factor is derived. For example, a mark out of 10 could be given for each result, for example one mark for the noise level, one mark for the content, and an average thereof is calculated. This will constitute the dataset-related component of the quality factor.

However, the quality factor always relates to a pair of medical dataset and evaluation method. Therefore, the evaluation method also contributes to the quality factor. To do this, in an embodiment, the system accesses a list of pre-determined merit factors of the available evaluation methods. Such merit factors can for example be determined by an independent organisation, such as the American Society of Echocardiography. For example, a left ventricular function which has been determined by a 3D-method will have a higher merit factor than the left ventricular function measured by monoplane or biplane Simpson's method. These merit factors evidently need not to be calculated afresh, but are accessed in the form of a pre-determined list.

According to the third point mentioned above, the quality factor may yet be influenced by further information, which is usually available in the form of stored data, and is usually not directly calculated from either the medical dataset or the specific evaluation method. For example, the further information may be related to the clinical question. These can be, for example, the skill of the user, as already mentioned above. A further kind of information which may be used is a database of clinical a priori knowledge. For example, a system needs to know if more than one kind of evaluation method has to be carried out to answer a specific clinical question. For example, intra-ventricular dissynchrony requires the analysis of both the ejection fraction and of the ECG. Another example of clinical a priori knowledge would be the fact that certain evaluation methods do not work well in diseased hearts. For example, the ejection fraction can well be measured with monoplane Simpson's method in reasonably normal hearts, but can give very poor results if the patient is suffering from severe heart failure. Therefore, the clinical a priori knowledge may comprise the fact that the NYHA-class has to be consulted before selecting an evaluation method, and if the NYHA -class is poor, 3D evaluation methods will be given preference.

Further information which may contribute to the quality factor is already available measurement data, for example, the NYHA -class of the patient or for example an already completed analysis of an ECG.

Another part of the further information which can contribute to the quality factor is the age of the patient. It may be known that certain evaluation tools do not work well on aged patients. Finally, further information may also be the type of device on which the evaluation method is to be carried out. If the device for example has no possibility of user interaction, all evaluation methods requiring such user interaction will automatically receive a quality factor of 0. Alternatively, the type of device may influence the speed with which the evaluation can be carried out. If the device has low computing capacity, 3D evaluation methods should be avoided and therefore receive low quality factors.

In an embodiment, the quality factor is first calculated by combining the merit factor related to the evaluation method, and the component related to the medical dataset. This may for example be done by simply multiplying the merit factor with the component related to the medical dataset, or by calculating an average. This provisional quality factor is then further modified, or not, depending on the further information such as clinical a priori knowledge, patient age, available measurement data and/or user skill. The user skill, for example, should only influence the quality factor if the specific evaluation tool requires user interaction.

The clinical a priori knowledge and the available measurement data may also be used during the selection step (f) and/or during the evaluation step (g).

In an embodiment, the analysis step d) may also comprise a step of preselecting one or several evaluation methods according to a pre-determined list of pre-selected evaluation methods. The pre-selection may be done by the clinical institution if some evaluation methods are preferred, for example in order to better compare the results between different patients.

In an embodiment, the analysis step comprises one or several of the following sequential steps: Optionally, those medical datasets which are generally suitable for returning relevant information with regard to the clinical question are pre-selected. This may be done, for example, by extracting the type of dataset (image dataset, ECG, etc.), the imaging modality (MRI, CT, Ultrasound) and the imaging mode (For Ultrasound: e.g. Doppler, B-mode, M-mode and the images sector) from each medical dataset, for example from the header or by OCR. In an embodiment, the above-described pre-analysis is carried out on all medical datsets. Thereby, a subset of the available medical dataset is pre-selected and further analysed.

The pre-selected (or all) medical datasets are then loaded into a working memory for further analysis.

Further analysis is then carried out, such as evaluation of the image content, the contrast profile, the image quality in terms of noise, etc. The further analysis will preferably comprise the more time-intensive operations of the above described steps, and may also comprise accessing the merit factors of the available evaluation methods and taking into account the further information.

Part of the analysis step may also be the modification of an image dataset to make it suitable for the available or preferred evaluation method(s). In particular, partial datasets may be extracted from a medical dataset. For example, if the analysis step finds out that a 2D evaluation method such as biplane Simpson's is the preferred evaluation method, but only a 3D dataset is available, the system may extract the two required 2D image slices, namely two long axis views of the ventricle, from the 3D image dataset. If such modification of at least one medical dataset has been carried out, the further analysis and calculation of a quality factor will be performed on the modified medical dataset.

In an embodiment, the component of the quality factor related to the medical datasets is combined with the merit factors of each evaluation tool, and optionally the further information mentioned above (user skill, patient age, etc.). The result can be outputted in the form of a quality factor for each pair of evaluation tool and medical dataset or modified medical dataset. One of these combinations will be selected as the best. Alternatively, if one of the evaluation methods is pre-selected as preferred, the system will output the medical dataset which is most suitable for this preferred evaluation tool. Optionally, the user may select one combination of dataset/evaluation tool.

In one embodiment, the evaluation tool selected automatically or by the user is then carried out. In an embodiment, the evaluation method may comprise the use of already available measurement values for the patient. For example, if the length of the QRS complex has already been determined previously, the evaluation method may perform the calculation of the left ventricular ejection fraction and may yield information on intraventricular dissynchrony.

The invention is also directed to a computer program adapted for performing the inventive method, when the computer program is executed on a computer. The computer program may comprise software code portions which induce a computer to carry out the method. As part of the method, the computer may also prompt the user to make selections or interact where necessary, for example to perform the evaluation method. For example, monoplane Simpson's method may require the user to draw in the end-diastolic and end-systolic ventricle contours.

The invention is further directed to a digitally readable medium such as a computer hard disk, a CD-Rom or a DVD, on which the above-described computer program is stored. The invention is also directed to a computer program product.

Further, the invention may also be embodied in a device (also called system above) for performing the inventive method. Such device must comprise at least a storage device for storing the one or several medical datasets, and a computing device for performing the analysis and selection steps. The actual evaluation step may be carried out by a different device or by the same device. The device may be an ordinary computer such as a PC or workstation. It may be connected to a PACS or hospital information system (HIS) for accessing the medical datasets of a particular patient. The device may also be a computer integrated into an ultrasound machine or another medical imaging device.

The invention shall now be described in relation to specific embodiments with reference to the appended drawings. In the drawings:
- Fig. 1: is a flow chart showing an overview of a method according to an embodiment of the invention;
- Fig. 2: is a flow chart demonstrating the input to the analysis step according to a first embodiment;
- Fig. 3: is a flow chart showing the input to the analysis step according to a second embodiment;
- Fig. 4: is a flow chart showing the analysis step (d) in more detail:
- Fig. 5: is a schematic representation of a device according to an embodiment of the invention.

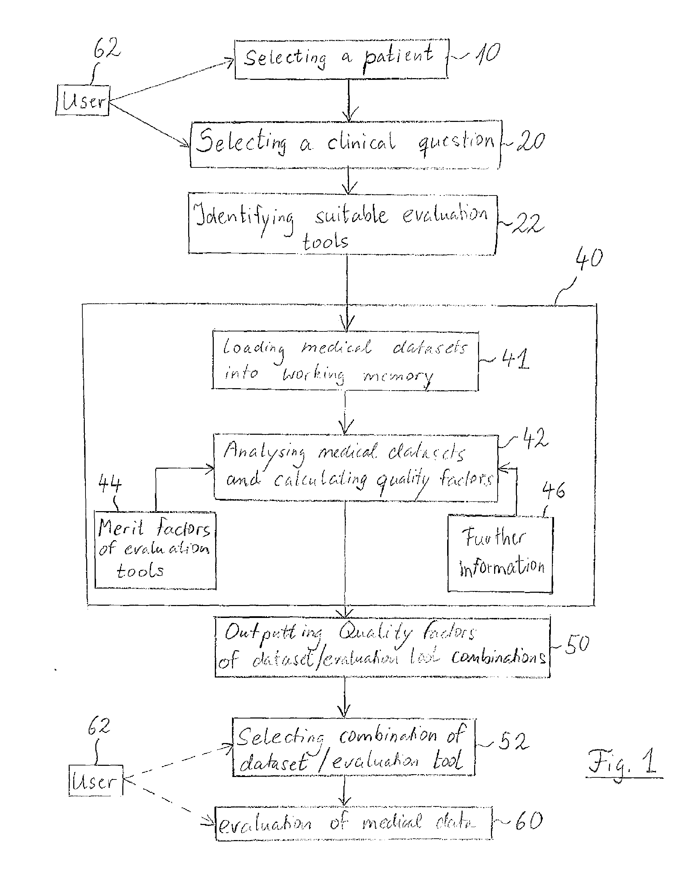

Fig. 1 shows a general overview of an embodiment of the inventive method.

In step 10, a patient is selected by the user 62, and in step 20, a clinical question is selected by the user 62. For example, the user may type in the patient name or receive it from a DICOM modality work list and, before or afterwards, will select the medical question from a list of available clinical questions. In one embodiment, all clinical questions are related to the heart and circulation.

The system will then automatically identify suitable evaluation tools related to the clinical question in step 22. This is usually done by accessing a pre-determined list listing all available evaluation tools for each clinical question. This list may also contain information if one of the available evaluation tools is preferred by the clinical institution.

The next steps are all part of the automatic analysis step 40. First, the medical datasets which are available for this patient are loaded into a working memory in step 41. As described above, previous to this step the system may pre-select those medical datasets which are generally suitable for returning relevant information with regard to the clinical question.

In step 42, the up-loaded medical datasets are analysed and corresponding quality factors are calculated. The quality factors may incorporate pre-determined merit factors of evaluation tools, and further information unrelated to the specific medical datasets, such as patient age, user skill, etc., as mentioned above.

The result of the analysis step 40 is a number of quality factors for each pair of medical dataset/evaluation tool. These quality factors are preferably outputted in step 50.

In the next step 52, a combination of medical dataset and evaluation tool is selected. This may be done either automatically by the system selecting the pair with the best quality factor. Alternatively, the selection may be done by the user, but of course being aware of the associated quality factor.

Finally, in step 60, the selected evaluation tool is carried out on the selected medical data. This evaluation may or may not require interaction by a user 62. The dotted line in Fig. 1 indicates that user interaction may or may not take place.

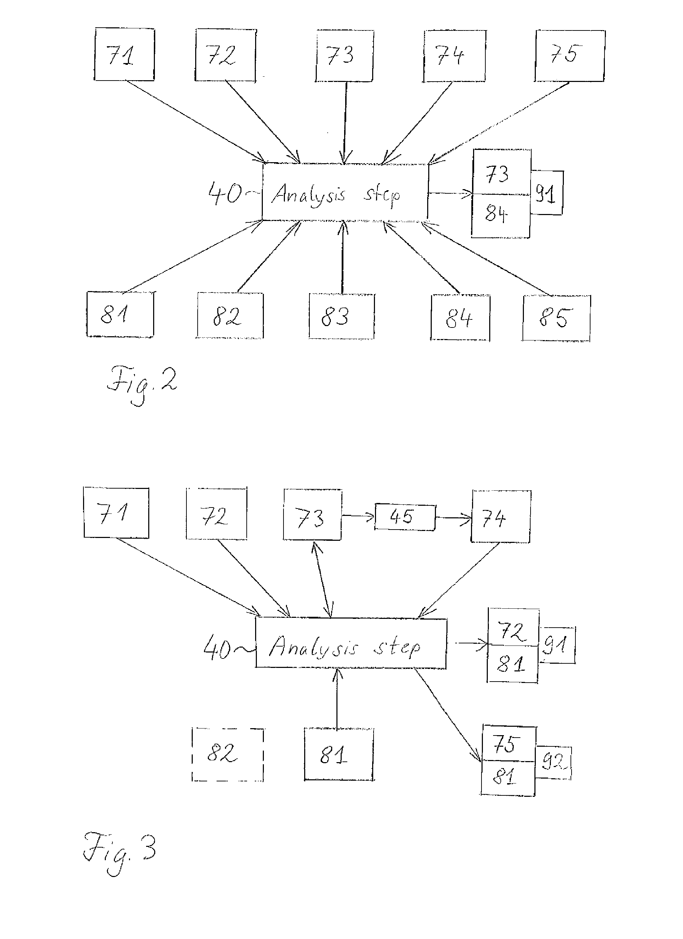

Fig. 2 and 3 illustrate two alternative ways in which the pair of medical dataset and evaluation method may be selected. Fig. 2 concerns a case in which several medical datasets 71, 72,...75 and several evaluation tools 81, 82,...,85 are available. All of these are inputted into the analysis step 40, and a quality factor is calculated for each pair of medical dataset and evaluation method. Thus, in total 5 x 5 = 25 quality factors are calculated. To reduce this number, the system may pre-select certain medical datasets prior to calculating the quality factors. In addition or alternatively, the system may pre-select one or several evaluation methods according to a pre-determined list of pre-selected evaluation methods.

Independent of any pre-selection, the system will output at least the pair of medical dataset and evaluation method having the best quality factor. In the present case, this will be medical dataset 73 if evaluated with evaluation tool 84, which is given the quality factor 91. Alternatively, the system may output the combinations having the best 2, 3 or 4 quality factors, or may simply output all calculated quality factors, thus giving the user a comprehensive overview over his choices of datasets and evaluation tools.

Fig. 3 concerns a slightly different case, namely where one evaluation method 81 is pre-selected as preferred. This may be done by the clinical institution to ensure that all clinical questions are handled by the same evaluation method 81, even if other evaluation methods 82 are theoretically also possible for this clinical question. In this case, the analysis step 40 simply analyses the medical datasets 71, 72, 73 for their suitability with regard to the one preferred evaluation method 81, and outputs respective quality factors 91, 92. Again, the system may output only the best quality factor(s) or all quality factors.

Another aspect of the analysis step is also illustrated in Fig. 3. Namely, if the analysis of one dataset 73 results in the finding that this dataset is not suitable to be evaluated with the preferred evaluation method 81, the system may modify the dataset 73 in the modification step 45, to result in another dataset 74, which is subjected to further analysis. The modification 45 may for example be the extraction of partial datasets from a medical image dataset.

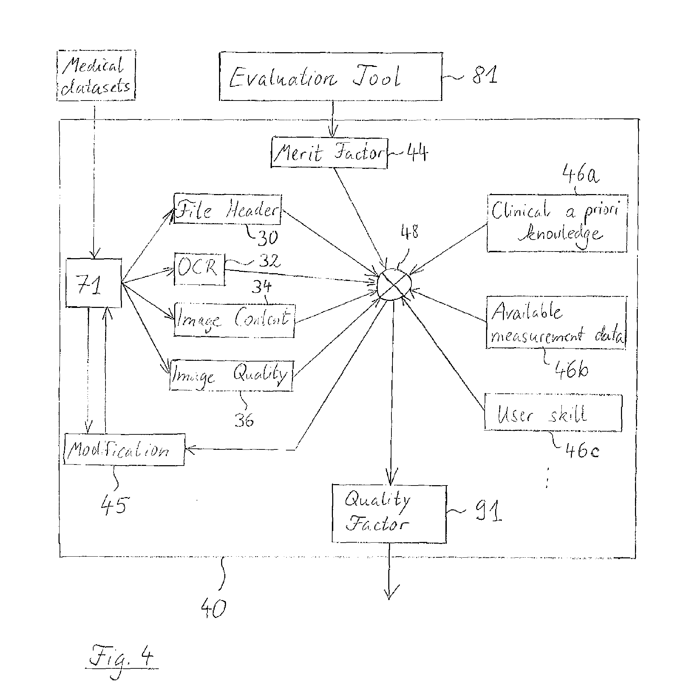

Fig. 4 shows the analysis step 40 in more detail. In particular, it should describe what exactly is happening to one medical dataset 71, when tested with regard to its suitability to be used for one particular evaluation tool 81.

The medical dataset 71 is analysed in order to extract certain information from it. In particular, the file header 30 may be scanned, or the image may be subjected to OCR 32, to extract information such as the type of medical dataset, imaging modality, the covered sector or field of view, spatial resolution, etc. (see above). Optionally, this extracted information may be used to either pre-select the dataset 71 for further use, or discard it as not suitable.

In addition, the medical dataset 71 may be scanned for image content 34 and/or image quality 36, for example image quality in terms of image contrast and/or image noise.

The extracted information is collected in the quality factor calculation unit 48. This unit (which is preferably a piece of software) controls the analysis step, and combines the collected information to calculate the resulting quality factor 91. It may, for example, conclude from the file header information 30 that the medical dataset 71 is not directly suitable for the evaluation tool 81, but may be made suitable by modifying the medical dataset in some way. In this case, the control unit 48 will initiate the necessary modification step 45, which will return a modified medical dataset 71. This may then be subjected to further analysis in terms of image content 34 and image quality 36.

The evaluation tool 81 has a pre-determined merit factor 44, which is also inputted into the control unit 48.

Optionally, further information may be inputted into the control unit 48, such as clinical a priori knowledge 46a, already available measurement data 46b from the patient, or a measure of user skill 46c. The already available measurement data 46b may for example be the NYHA-class of the patient, the patient age, results of ECG measurements and results of previous evaluations, possibly with the same evaluation tool 81.

All this information is collected in a control unit 48 and used to calculate quality factor 91 connected to the particular combination of medical dataset 71 and evaluation tool 81. The quality factor may be a multiplication of the merit factor with the contribution related to the medical dataset, possibly modified by further information, or it may be the average of different contributions, etc. How exactly the quality factor is calculated will depend on the priorities of the specific clinical institution, but it is expected that standards will evolve with time.

The quality factor 91 is then outputted and further used according to Fig. 1.

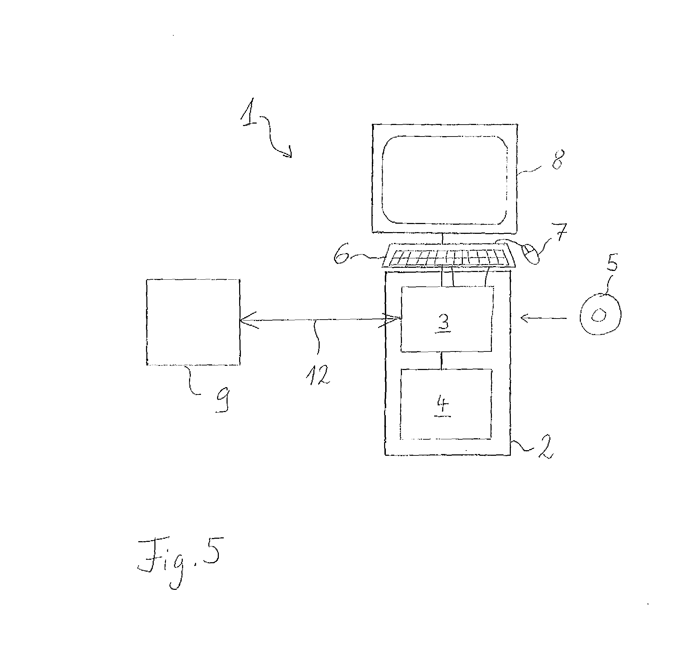

Fig. 5 is a schematic drawing of a device 1 with which the present invention can be carried out. The device 1 may comprise a computer 2 comprising at least a processing unit 3 such as a CPU, and a storage 4. The data storage 4 may be a random access memory (RAM). In addition, a hard disk and other storage means may be present. The device may also be part of the imaging modality itself, e.g. the CPU of an ultrasound system.

The method may be installed on the device 1 by means of a digitally readable medium such as a DVD 5 or via network connection. As means for user interaction, the computer 2 is connected to input devices such as a keyboard 6, a mouse 7 and one or several displays 8.

The medical datasets of the patient can be stored on the computer 2. Preferably, however, they are stored on a multimodal platform 9 such as a PACS, CVIS, RIS or Hospital Information System (HIS), which is accessed via a network connection 12, for example a local area network or via the internet. In this case, suitable encryption techniques should be used to ensure patient privacy. The user of the device 1 will usually be medical personnel, such as doctors and medical, technical and radiology assistants. By the inventive method, the user is spared from tediously going through a multitude of medical datasets to select the best images or medical datasets for further evaluation.

## Claims

1. A method for analysing medical data in view of a specific clinical question, the method comprising the following steps:
e) providing one or several medical datasets (71-75) comprising data acquired by means of one or several diagnostic modalities from one patient;
f) providing (20) a clinical question to be answered by the medical dataset(s);
g) selecting (22) one or several evaluation methods (81-85) which are generally suitable for returning relevant information with regard to the clinical question;
h) automatically analysing (40) the medical dataset(s) with regard to their suitability to be used for the one or several evaluation methods (81-85) and calculating corresponding quality factors (91, 92) for each pair of medical dataset and evaluation method.

2. The method according to claim 1, further comprising the step of
e) outputting (50) the calculated quality factors.

3. The method according to claim 1 or 2, further comprising the step of
f) selecting (52) at least one medical dataset (71-75) as suitable to be evaluated by at least one evaluation method (81-85), the selection consisting of:
- on the basis of the calculated quality factors, selecting one evaluation (84) method for at least one of the medical dataset(s) (73); and/or
- on the basis of the calculated quality factors, selecting one of several medical datasets (71-73) to be evaluated by one preferred pre-selected evaluation method (81).

4. The method according to claim 1, 2 or 3, further comprising the steps of:
g) evaluating (60) the selected at least one medical dataset by the selected or the preferred evaluation method;
h) outputting the result of the evaluation method; and
i) outputting the quality factor associated with the outputted result.

5. The method of claim 1, 2 or 3, further comprising the steps of:
g) evaluating (60) the selected at least one medical dataset by the preferred evaluation method and, in addition, by one or more of the other generally suitable evaluation methods;
h) outputting the results of each evaluation method; and
i) outputting the quality factor associated with each outputted result.

6. The method according to any one of the preceding claims, wherein the one or several medical datasets (71-75) are selected from the group comprising:
● image datasets, such as two-dimensional (2D), three-dimensional (3D) or four-dimensional (4D) image datasets acquired by means of Ultrasound, Computed Tomography (CT), Magnetic Resonance Imaging (MRI), Infrared or X-Ray;
● electromedical datasets such as ECG-datasets or EEG-datasets;
● hemodynamic datasets, and
● blood pressure datasets.

7. The method according to any one of the preceding claims, wherein the one or several evaluation methods (81-85) may comprise:
● 2D evaluation methods for image datasets;
● 3D evaluation methods for image datasets;
● 4D evaluation methods for image datasets;
● measurement of the synchrony of the movement of a heart chamber;
● measurement of the cross section of a blood vessel;
● measurement of the intima-media thickness (IMT) of a blood-vessel;
● measurement of the degree of stenosis of a blood vessel;
● measurement of muscle deformation such as strain, displacement velocity, thickening;
● determination of a ventricular ejection fraction by means of single plane Simpson's method to measure the ventricular volume;
● determination of a ventricular ejection fraction by means of biplane Simpson's method to measure the ventricular volume;
● determination of a ventricular ejection fraction by means of 3D volume measurements of the ventricular volume.

8. The method according to any one of the preceding claims, wherein the clinical question is related to the cardiovascular system and in particular is selected from the group comprising:
● determining the risk for a vascular disease;
● determining the risk for a heart disease;
● determining whether the patient has a coronary heart disease;
● determining the ventricular function, e.g. the ejection fraction;
● determining whether an intraventricular dissynchrony is present;
● determining the valvular function.

9. The method according to any one of the preceding claims, wherein the analysis step d) comprises one or several of the following steps related to the analysis of the provided medical dataset(s):
● the readout of data included in the file header(s) of the one or several medical datasets (30);
● OCR (32) of text data on a digital image;
● extracting one or more of the imaging modality, the acquisition mode, the dimension of the dataset (2D, 3D; 3D), the time resolution, the spatial resolution and/or the field-of-view of an image dataset, preferably by readout of data contained in the file header(s) of the one or several image datasets or by OCR;
● determination of the content (34) of a medical image dataset;
● determination of the quality (36) of a medical image dataset in terms of the content of the image in view of the medical question;
● determination of the contrast profile of a medical image dataset;
● determination of the quality (36) of an image dataset in terms of noise level;
● determination of the quality (36) of an image dataset in terms of spatial resolution and/or field-of-view;
● determining the type of a medical image dataset in terms of imaging modality, 2D, 3D, 4D.

10. The method according to any one of the preceding claims, wherein the analysis step d) comprises accessing a list of pre-determined merit factors (44) for the available evaluation methods.

11. The method according to any one of the preceding claims, wherein the quality factor for each pair of medical dataset and evaluation method is calculated on the basis of one or both of the following:
● a component related to the medical dataset, in particular as determined by the analysis step of claim 12;
● a pre-determined merit factor of the evaluation method.

12. The method according to any one of the preceding claims, wherein the quality factor for each pair of medical dataset and evaluation method is calculated by accessing and taking into account of one or several of the following further information:
● already available measurement values (46b) for the patient;
● the skill of the user (46c);
● clinical a priori knowledge (46a) concerning the clinical question;
● the age of the patient;
● the type of device on which the evaluation is to be carried out.

13. The method according to any one of the preceding claims, wherein the analysis step d) comprises one or several of the following steps:
d1) optionally preselecting those medical datasets which are generally suitable for returning relevant information with regard to the clinical question;
d2) loading the provided or preselected medical datasets into a working memory;
d3) performing an analysis on the medical datasets in the working memory to calculate a quality factor for each available or pre-selected evaluation method.

14. The method according to any one of the preceding claims, wherein the analysis step d) or the evaluation step f) comprises a step of modifying (45) the at least one medical dataset to make it suitable for the available or preferred evaluation method(s), in particular a step of extracting partial datasets from at least one medical dataset.

15. A computer program adapted for performing the method according to any one of the preceding claims, when the computer program is executed on a computer.

16. A device for performing the method according to any one of the preceding claims, comprising
● a storage device for storing the one or several medical datasets;
● a computing device for performing the analysis steps d).

## Amended claims

### Amended claims in accordance with Rule 137(2) EPC.

**1.** A method for analysing medical data in view of a specific clinical question, the method comprising the following steps:
a) providing one or several medical datasets (71-75) comprising data acquired by means of one or several diagnostic modalities from one patient;
b) providing (20) a clinical question to be answered by the medical dataset(s);
c) providing (22) one or several evaluation methods (81-85) for analysing medical data which are generally suitable for returning relevant information with regard to the clinical question;
d) automatically analysing (40) the medical dataset(s) with regard to their suitability to be used for the one or several evaluation methods (81-85) and calculating corresponding quality factors (91, 92) for each pair of medical dataset and evaluation method.

**2.** The method according to claim 1, further comprising the step of e) outputting (50) the calculated quality factors.

**3.** The method according to claim 1 or 2, further comprising the step of f) selecting (52) at least one medical dataset (71-75) as suitable to be evaluated by at least one evaluation method (81-85), the selection consisting of:
- on the basis of the calculated quality factors, selecting one evaluation (84) method for at least one of the medical dataset(s) (73); and/or
- on the basis of the calculated quality factors, selecting one of several medical datasets (71-73) to be evaluated by one preferred pre-selected evaluation method (81).

**4.** The method according to claim 1, 2 or 3, further comprising the steps of:
g) evaluating (60) the selected at least one medical dataset by the selected or the preferred evaluation method;
h) outputting the result of the evaluation method; and
i) outputting the quality factor associated with the outputted result.

**5.** The method of claim 1, 2 or 3, further comprising the steps of:
g) evaluating (60) the selected at least one medical dataset by the preferred evaluation method and, in addition, by one or more of the other generally suitable evaluation methods;
h) outputting the results of each evaluation method; and
i) outputting the quality factor associated with each outputted result.

**6.** The method according to any one of the preceding claims, wherein the one or several medical datasets (71-75) are selected from the group comprising:
• image datasets, such as two-dimensional (2D), three-dimensional (3D) or four-dimensional (4D) image datasets acquired by means of Ultrasound, Computed Tomography (CT), Magnetic Resonance Imaging (MRI), Infrared or X-Ray;
• electromedical datasets such as ECG-datasets or EEG-datasets;
• hemodynamic datasets, and
• blood pressure datasets.

**7.** The method according to any one of the preceding claims, wherein the one or several evaluation methods (81-85) may comprise:
• 2D evaluation methods for image datasets;
• 3D evaluation methods for image datasets;
• 4D evaluation methods for image datasets;
• measurement of the synchrony of the movement of a heart chamber;
• measurement of the cross section of a blood vessel;
• measurement of the intima-media thickness (IMT) of a blood-vessel;
• measurement of the degree of stenosis of a blood vessel;
• measurement of muscle deformation such as strain, displacement velocity, thickening;
• determination of a ventricular ejection fraction by means of single plane Simpson's method to measure the ventricular volume;
• determination of a ventricular ejection fraction by means of biplane Simpson's method to measure the ventricular volume;
• determination of a ventricular ejection fraction by means of 3D volume measurements of the ventricular volume.

**8.** The method according to any one of the preceding claims, wherein the clinical question is related to the cardiovascular system and in particular is selected from the group comprising:
• determining the risk for a vascular disease;
• determining the risk for a heart disease;
• determining whether the patient has a coronary heart disease;
• determining the ventricular function, e.g. the ejection fraction;
• determining whether an intraventricular dissynchrony is present;
• determining the valvular function.

**9.** The method according to any one of the preceding claims, wherein the analysis step d) comprises one or several of the following steps related to the analysis of the provided medical dataset(s):
• the readout of data included in the file header(s) of the one or several medical datasets (30);
• OCR (32) of text data on a digital image;
• extracting one or more of the imaging modality, the acquisition mode, the dimension of the dataset (2D, 3D; 3D), the time resolution, the spatial resolution and/or the field-of-view of an image dataset, preferably by readout of data contained in the file header(s) of the one or several image datasets or by OCR;
• determination of the content (34) of a medical image dataset;
• determination of the quality (36) of a medical image dataset in terms of the content of the image in view of the medical question;
• determination of the contrast profile of a medical image dataset;
• determination of the quality (36) of an image dataset in terms of noise level;
• determination of the quality (36) of an image dataset in terms of spatial resolution and/or field-of-view;
• determining the type of a medical image dataset in terms of imaging modality, 2D, 3D, 4D.

**10.** The method according to any one of the preceding claims, wherein the analysis step d) comprises accessing a list of pre-determined merit factors (44) for the available evaluation methods.

**11.** The method according to any one of the preceding claims, wherein the quality factor for each pair of medical dataset and evaluation method is calculated on the basis of one or both of the following:
• a component related to the medical dataset, in particular as determined by the analysis step of claim 12;
• a pre-determined merit factor of the evaluation method.

**12.** The method according to any one of the preceding claims, wherein the quality factor for each pair of medical dataset and evaluation method is calculated by accessing and taking into account of one or several of the following further information:
• already available measurement values (46b) for the patient;
• the skill of the user (46c);
• clinical a priori knowledge (46a) concerning the clinical question;
• the age of the patient;
• the type of device on which the evaluation is to be carried out.

**13.** The method according to any one of the preceding claims, wherein the analysis step d) comprises one or several of the following steps:
d1) optionally preselecting those medical datasets which are generally suitable for returning relevant information with regard to the clinical question;
d2) loading the provided or preselected medical datasets into a working memory;
d3) performing an analysis on the medical datasets in the working memory to calculate a quality factor for each available or pre-selected evaluation method.

**14.** The method according to any one of the preceding claims, wherein the analysis step d) or the evaluation step f) comprises a step of modifying (45) the at least one medical dataset to make it suitable for the available or preferred evaluation method(s).

**15.** A computer program adapted for performing the method according to any one of the preceding claims, when the computer program is executed on a computer.

**16.** A device for performing the method according to any one of the preceding claims, comprising
• a storage device for storing the one or several medical datasets;
• a computing device for performing the analysis steps d).
